# FASCICULE DE BREVET EUROPEEN

(11) **EP 0 865 487 B1**
(45) Date de publication et mention de la délivrance du brevet: **04.08.2004**
(21) Numéro de dépôt: 96941701.3
(22) Date de dépôt: 04.12.1996
(51) Int. Cl.: C12N 15/10, C12N 15/62, C07K 16/10, C12N 7/01, C07K 16/00

(54) **PREPARATION DE BANQUE MULTICOMBINATOIRE DE VECTEURS D'EXPRESSION DE GENES D'ANTICORPS**
HERSTELLUNG EINER MULTIKOMBINATONISCHEN BANK VON ANTIKÖRPERGENEN EXPRIMIERENDENVEKTOREN
PREPARATION OF A MULTICOMBINATORIAL LIBRARY OF ANTIBODY GENE EXPRESSION VECTORS

(30) Priorité: 04.12.1995 FR 9514325
(43) Date de publication de la demande: 23.09.1998
(73) Titulaire: Aventis Pasteur, 69367 Lyon Cedex 07 (FR)
(72) Inventeur: SODOYER, Régis, F-69110 Sainte-Foy-lès-Lyon (FR); AUJAME, Luc, F-69210 Fleurieux-sur-l'Arbresle (FR); GEOFFROY, Frédérique, F-69690 Bessenay (FR); BOUCHARDON, Annabelle, F-69007 Lyon (FR)
(74) Mandataire: Bernasconi, Jean Raymond
(86) Numéro de dépôt international: PCT/FR1996/001938
(87) Numéro de publication internationale: WO 1997/020923

(56) Documents cités:
- WO-A-92/20791
- WO-A-93/19172
- WO-A-95/21914
- WO-A-96/07754
- GENE, vol. 151, no. 1-2, 1994, AMSTERDAM NL, pages 109-113, XP000579650 GEOFFROY, F. ET AL.: "A new phage system to construct multicombinatorial libraries of very large antibody repertoires"
- 9TH INTERNATIONAL CONGRESS OF IMMUNOLOGY, vol. 0, no. 0, 1995, SAN FRANCISCO, USA, page 464 XP000600281 SODOYER, R. ET AL.: "A new phage-display system to construct multicombinatorial libraries of very large antibody repertoires" & 9th International Congress of Immunology San Francisco, USA 23 au 29 Juillet 1995
- CELL, vol. 59, no. 1, 6 Octobre 1989, NA US, pages 197-206, XP000579648 NUNES-DÜBY, S.E. ET AL.: "Half -att site substrate reveal the homology independence and minimal protein requirements for productive synapsis in Lambda excisive recombination"
- EMBO JOURNAL, vol. 12, no. 12, 1 Décembre 1993, EYNSHAM, OXFORD GB, pages 4567-4576, XP000579649 SEGALL, A.M. & NASH H.A.: "Synaptic intermediates in bacteriophage lambda site-specific recombination: integrase can align pairs oh attachment sites"
- SCIENCE, vol. 256, no. 5054, 10 Avril 1992, LANCASTER, PA US, pages 198-203, XP000579651 KIM, S. & LANDY, A.: "Lambda Int protein bridges between higher order complexes at two distant chromosomal loci attL and attR"

## Description

Les molécules d'anticorps sont constituées par l'association de deux chaînes lourdes (H) et de deux chaînes légères (L) par l'intermédiaire de ponts disulfure. Les deux chaînes lourdes sont associées entre elles selon une structure en forme de Y et les deux chaînes légères s'associent respectivement aux deux branches de cette structure, de façon telle que les domaines variables des chaînes légères (V_{L}) et lourdes (V_{H}) soient situés à proximité l'un de l'autre. La liaison à l'antigène résulte des propriétés des parties variables des chaînes légères et lourdes. Un système complexe de réarrangement et de sélection permet alors d'induire rapidement une quantité importante d'anticorps spécifiques contre un antigène.

La technique classique des hybridomes permet la sélection de clones de cellules hybrides exprimant des gènes codant pour les chaînes légère et lourde d'une molécule d'anticorps. Cette technique nécessite la fusion de cellules d'origine lymphocytaire, contenant les gènes présidant à la formation des anticorps et de cellules formant des lignées immortalisées. Les cellules portant les gènes en question sont en général obtenues par création au hasard de banques de cellules circulantes, avec ou sans immunisation préalable par l'antigène spécifique, et le criblage des hybridomes s'effectue par une réaction antigène-anticorps après multiplications et cultures des clones hybridomes. Cette technique est lourde, d'un rendement limité et le criblage n'est pas facile.

Une autre méthode, utilisant des bactériophages recombinants a été récemment mise en oeuvre. Les principes et diverses réalisations de ce procédé sont par exemple décrits par D. R. Burton, Tiptech - mai 1991, vol. 9, 169-175 ; D. J. Chiswell et al., Tiptech - mars 1992, vol. 10, 80-84 ; H. R. Hoogenboom et al., Rev. Fr. Transfus. Hémobiol., 1993, 36, 19-47 (voir également les demandes de brevets PCT WO 92/01047 et 92/20791).

Cette technique consiste à insérer, dans un vecteur, un répertoire de gènes de régions variables d'anticorps en association avec un gène de bactériophage dans des conditions permettant l'expression du gène sous la forme d'une protéine de fusion se présentant à la surface du phage, exposant les régions variables des chaînes variables légère et lourde associées par leurs ponts disulfure à la façon d'un fragment Fab d'anticorps, et à sélectionner directement les phages par une méthode rapide de séparation utilisant l'antigène spécifique immobilisé, par exemple par chromatographie d'immuno-affinité. Les phages sélectionnés, après élution, peuvent infecter une bactérie et être utilisés pour une production directe ou pour répéter des cycles de sélection. Cette méthode est particulièrement puissante car elle permet la création, en théorie, de banques très importantes et un criblage extrêmement fin, efficace et rapide de la banque. Un phage qui se prête particulièrement bien à ce procédé est le phage filamenteux fd, dans lequel on peut fusionner le fragment codant pour l'une des chaînes lourde et légère de l'anticorps avec le gène de la protéine mineure de surface et insérer le fragment codant pour l'autre chaîne, de sorte que, après infection de bactéries par le phage, on obtient une population de phages portant à leur surface une protéine de fusion présentant les chaînes lourde et légère dans une configuration capable de reconnaître l'antigène, et donc appropriée au criblable.

Outre sa simplicité, cette technique présente de grands avantages. En association avec l'amplification préalable de la banque de gènes d'anticorps, on peut arriver à sélectionner un phage présentant un fragment d'anticorps spécifique dans une très grande population de phages, de l'ordre de 10⁷, ce qui peut permettre de rechercher les gènes d'anticorps humains sans avoir forcément à procéder à l'immunisation préalable du donneur.

Par clivage suivi de religation, ou à partir de deux banques séparées de gènes de chaînes légères et de chaînes lourdes on peut obtenir des phages associant une chaîne légère et une chaîne lourde au hasard.

Le nombre de clones différents que l'on peut obtenir est cependant limité par le rendement de la sélection et par le degré d'efficacité de la transformation bactérienne.

Un moyen d'accroître le nombre d'associations réussies associant les chaînes légères d'une première banque aux chaînes lourdes d'une deuxième banque été décrit par P. WATERHOUSE et al., Nucleic acids Research, 1993, Vol. 21, No. 9, 2265-2266. Il permet d'obtenir jusqu'à 10¹² clones, en utilisant un système de recombinaison spécifique de sites loxP sensible à l'action d'une recombinase Cre. Cependant l'association reste réversible. De plus il n'existe aucun contrôle de l'action de la recombinase et les vecteurs recombinés n'ont aucun avantage sélectif par rapport aux autres vecteurs.

Or, compte-tenu du rendement de l'étape de sélection des phages recombinés, qui, en réalité, ne retient qu'une fraction des phages intéressants, il est souhaitable d'obtenir les plus hauts rendements possibles de vecteurs recombinés avec le moins possible de vecteurs non-recombinés.

Dans le cadre d'une demande précédente (WO 95/21914 déposée le 2 février 1995 sous la priorité française FR 94 01519 du 10 février 1994) les auteurs de la présente invention ont décrit un procédé de production de banques multicombinatoires, et notamment sous forme de phages ou phagemides, à partir de deux répertoires de gènes, l'un de chaînes légères et l'autre de chaînes lourdes, permettant d'obtenir un nombre de clones élevé.

Ce système possède des propriétés de non reversibilité et de sélectivité améliorées par le fait qu'un nouveau marqueur de sélection apparaît après recombinaison.

Les propriétés de non réversibilité sont dues à l'absence de moyens d'excision, aussi bien dans les vecteurs que dans la souche-hôte. Les exemples illustrant cette demande se caractérisent ainsi par l'absence de la protéine d'excision Xis aussi bien dans les vecteurs que dans la souche Xis⁻. D'autre part, le caractère stable des séquences de jonction issues de la recombinaison des sites de recombinaison spécifiques contribue à la non réversibilité du système.

La présente invention vise à perfectionner ce procédé, notamment à augmenter son rendement, sa facilité de mise en oeuvre ainsi que de la diversité de clones générés.

Dans cet objectif, l'invention permet de réaliser après deux événements de recombinaison, un échange de séquences entre les deux vecteurs. Cet échange donne lieu à deux vecteurs recombinants, dont l'un possède les deux unités de transcription pour les chaînes lourde et légère mais dont la taille est inférieure à celle d'un vecteur qui résulterait d'une fusion entre les deux vecteurs de départ. Le vecteur final étant plus petit, sa réplication et son empaquetage sont plus efficaces et la production en est d'autant améliorée, ce qui augmente également le nombre de clones finaux.

L'invention permet en outre d'élargir considérablement le choix des souches utilisables en tant que cellules hôtes, dans la mesure où il n'est plus nécessaire que la souche utilisée possède dans son génome le gène de l'intégrase, ce gène étant à présent porté par l'un des deux vecteurs de départ et se retrouvant sur un vecteur final.

Ceci permet de s'affranchir des souches possédant le gène de l'intégrase intégré dans leur génome et de choisir toute souche qui soit hautement infectieuse et productrice de phages (TG1, 71-18 ou NM522).

Les souches 71-18 (Stratagène ; Yanisch-Perron, C et al. (1985) Gene 33, 103-109) et NM522 (New England Biolabs ; Woodcock, D.L. et al. (1989) Nucl. Acids. Res. 17, 1563-1575) se sont révélées particulièrement bonnes productrices de phages. Elles permettent une multiplication du nombre de phages produits d'un facteur de 10 à 50 par rapport notamment à la souche E. coli D1210HP diffusée par Stratagène.

L'invention a pour objet un procédé de production de banques multicombinatoires dans lequel, partant d'un premier répertoire de gènes codant pour une population d'un de deux types de polypeptides susceptibles de s'associer, de manière covalente ou non, notamment de régions variables de l'un des types chaîne légère et chaîne lourde d'anticorps, et d'au moins d'un gène codant pour l'autre type de polypeptide, notamment une région variable de l'autre type, de chaîne d'anticorps ou de préférence d'un second répertoire de gènes codants pour une population dudit autre type, on introduit les gènes dudit premier répertoire dans un premier vecteur pour former une population de vecteurs portant les différents gènes dudit premier répertoire, et on introduit ledit gène dudit autre type ou les gènes dudit second répertoire dans un second vecteur, et l'on procède à une recombinaison desdits premier et second vecteurs dans des conditions permettant à l'un des vecteurs de contenir, après recombinaison, un gène de l'un des deux types et un gène de l'autre type et d'exprimer les deux gènes sous forme de polypeptides associés susceptibles d'apparaître à la surface extérieure du produit dudit vecteur en y restant maintenus et en étant associés entre eux de façon multimérique ou simulant un multimère, caractérisé en ce que lesdits premiers et seconds vecteurs présentent des moyens pour échanger par recombinaison(s) irréversible(s) chacun une partie de façon à générer, après recombinaison(s) des vecteurs finaux recombinés dont l'un contient un gène de l'un des deux types et un gène de l'autre type.

De façon avantageuse le procédé selon l'invention est caractérisé en ce que les vecteurs de départ contiennent respectivement deux sites de recombinaisons spécifiques d'un ou de deux système(s) de recombinaison spécifique, notamment les sites attB de E. coli et attP du phage lambda, agencés de façon à permettre deux événements de recombinaison sous l'effet d'une recombinase ou intégrase associée pour former dans chacun des vecteurs finaux résultant de la recombinaison, deux séquences de jonctions stables telles que attL et attR.

Préférentiellement les deux sites de recombinaison compris dans chacun des vecteurs de départ sont en orientation inverse.

Dans une variante avantageuse, les vecteurs de départ contiennent respectivement deux sites attP du phage lambda et deux sites attB de E. coli.

De façon préférée l'un des deux vecteurs de départ comprend en outre une séquence codante pour une recombinase ou une intégrase. Cette variante avantageuse permet de pouvoir utiliser toute souche qui soit hautement infectieuse et productrice de phage, par exemple TG1, 71-18 ou NM522, sans devoir se limiter aux seules souches possédant dans leur génome le gène de l'une ou l'autre de ces enzymes. Les souches NM522 et 71-18 s'avèrent particulièrement intéressantes dans ce contexte d'utilisations. Préférentiellement l'enzyme que l'on utilise pour contrôler l'étape de recombinaison entre les vecteurs de départ est une recombinase inductible, et notamment une recombinase thermo-inductible.

Avantageusement, dans le procédé de l'invention le vecteur final recombinant obtenu à partir des vecteurs de départ qui contient les gènes à exprimer, est agencé pour présenter un marqueur de sélection initialement non fonctionnel et rendu fonctionnel par la recombinaison. Ce marqueur est par exemple un gène de résistance à un antibiotique, notamment un gène du groupe formé par les gènes de résistance aux tétracyclines, à la gentamycine, à la kanamycine et au chloramphenicol, avec son promoteur.

Préférentiellement ce marqueur de sélection comporte un promoteur propre au gène, le promoteur étant initialement inséré dans l'un des vecteurs de départ et le gène marqueur dans l'autre. Le gène de sélection et son promoteur peuvent être séparés par exemple par une séquence antiterminatrice, notamment la séquence NutL. Les vecteurs de départ contiennent également au moins une origine de réplication de phage ou une origine de replication de plasmide, lesdites origines étant agencées de manière à ce que les vecteurs finaux recombinants ne contiennent chacun qu'une origine de réplication de phage et/ou de plasmide.

Dans le procédé selon l'invention, l'un des deux gène à exprimer est fusionné à une séquence codant pour tout ou partie d'un polypeptide de la capside d'un phage. Cette séquence correspond par exemple au gène codant pour la protéine III du phage lambda. Avantageusement, ledit gène à exprimer et la séquence à laquelle il est fusionné sont situés dans un des vecteurs de départ de manière à être présents dans le produit phagemide recombinant contenant les deux gènes à exprimer.

L'invention a également pour objet les vecteurs obtenus ou susceptibles d'être obtenus par le procédé décrit ci-dessus, notamment les vecteurs de type plasmide ou phagemide qui se caractérisent par la présence d'une séquence codant pour l'un de deux types de polypeptides susceptibles de s'associer, notamment une partie variable de chaîne légère d'anticorps, et une séquence codant pour l'autre desdits deux types, notamment une partie variable de chaîne lourde d'anticorps, lesdites séquences étant accompagnées, dans des cadres convenables, des éléments permettant leur expression dans un hôte, lesdites séquences étant séparées par des séquences de jonction stables, notamment, attR et attL.

Ces vecteurs, notamment de type plasmide, comprennent preférentiellement une séquence fonctionnelle codant pour une recombinase ou une intégrase, et de façon particulièrement avantageuse ils comprennent également des séquences de jonction stable séparées par un espaceur.

L'invention a aussi pour objet des banques multicombinatoires de vecteurs, formées par les vecteurs selon l'invention et associant, de façon aléatoire, une séquence codant pour l'un de deux types de polypeptides susceptibles de s'associer, notamment une partie variable de chaîne légère d'anticorps et une séquence codant pour l'autre desdits deux types, notamment une partie variable de chaîne lourde d'anticorps.

L'invention a enfin pour objet les anticorps ou les parties d'anticorps de type Fab susceptibles d'être obtenus après sélection des banques de vecteurs selon l'invention à l'aide des marqueurs de sélection, puis criblage destiné à sélectionner les clones exprimant des associations de chaîne légère et chaîne lourde d'anticorps ayant les affinités recherchées contre les antigènes déterminés, puis expression des clones criblés dans un système d'expression, et enfin extraction et/ou purification des anticorps ou des parties d'anticorps de type Fab produits dans le système d'expression. Les techniques de sélection et de criblage sont par exemple décrites dans Parmley SF et al., Gené 73 (1988), 305-318. Les techniques d'extraction et de purification sont par exemple décrites dans Neu H.C. et al. ; (1965). J. Biol. Chem. 240, 3685-3692.

On décrit à présent un exemple de construction d'un vecteur recombiné selon l'invention combinant les parties variables de la chaîne légère et de la chaîne lourde d'un même clone anti-gp160 de HIV, vecteur qui s'avère capable d'exprimer les gènes desdites parties variables légères et lourdes et de présenter leurs produits d'expression à sa surface ou, en variante, de les relarguer sous forme d'une association chaîne lourde-chaîne légère reconnaissant l'antigène gp160.

La même technique pourra être utilisée pour réaliser les constructions multicombinatoires associant les gènes d'un répertoire de parties variables de chaîne légère à un gène de partie variable de chaîne lourde, ou un répertoire de parties variables de chaîne lourde à un gène de partie variable de chaîne légère, ou deux répertoires de parties variables lourdes et légères.

### Figures :

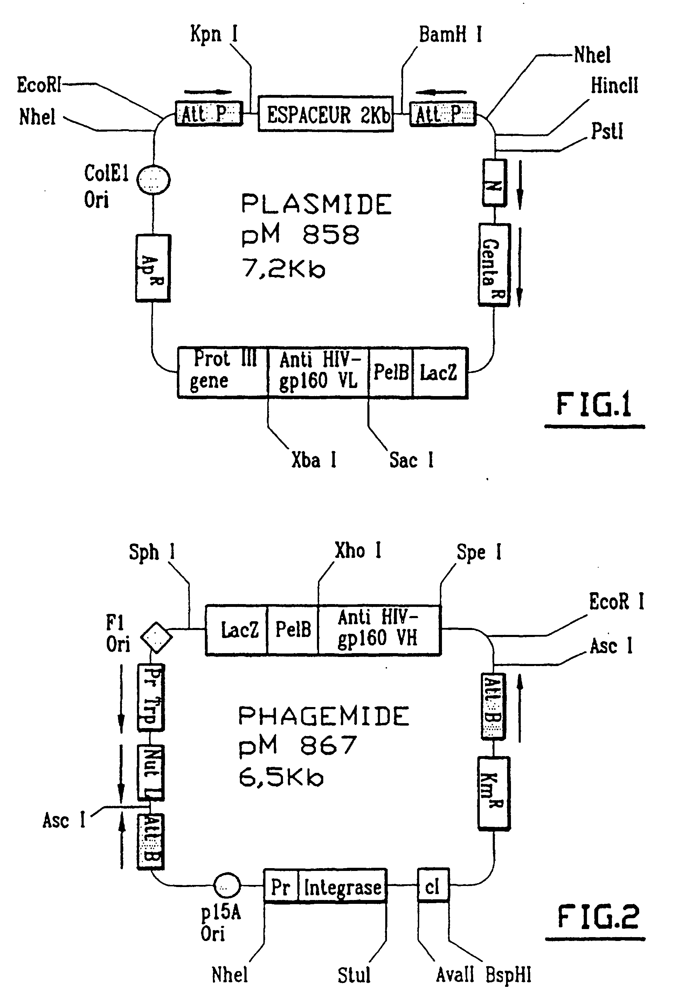

La figure 1 représente une vue schématique du plasmide pM858 possédant une unité de transcription pour les régions variables de chaînes légères en fusion avec le gène III.
La figure 2 représente une vue schématique du phagemide pM867 possédant une unité de transcription pour les régions variables de chaînes lourdes.

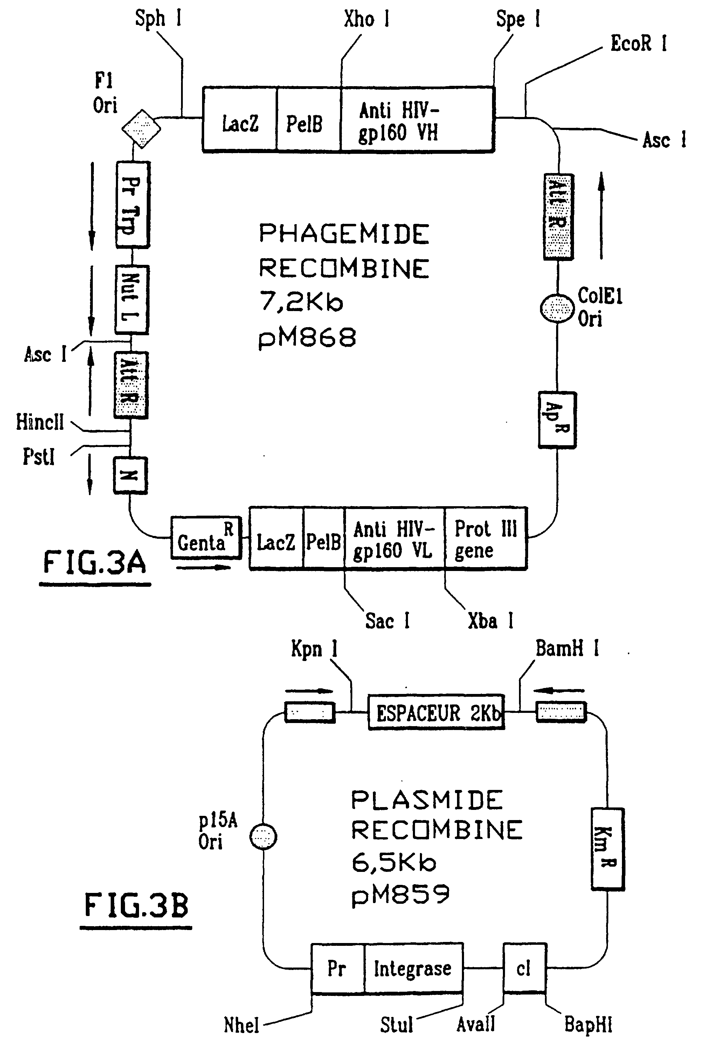

La figure 3 représente une vue schématique des vecteurs recombinants obtenus après échange de séquence entre le plasmide pM858 et le phagemide pM867.
La figure 3A représente le phagemide multicombinatoire de 7,2kb possédant les deux unités de transcription pour les chaînes lourde et légère.
La figure 3B représente le plasmide recombinant de 6,5kb possédant le gène de l'intégrase.

### Description des vecteurs :

Pour le détail des techniques de biologie moléculaire mises en oeuvre dans la construction des vecteurs on se référera à la demande WO 95/21914 incorporée ici par référence.

Plasmide pM858 (7.2kb) : Plasmide possédant l'origine de réplication "haut nombre de copies" Co1E1, une unité de transcription pour les régions variables de chaînes légères en fusion avec gène III (Promoteur lacZ-séquence signal PelB-VH gène III), le gène N suivi du gène codant pour le marqueur de sélection à la gentamycine, le marqueur de sélection à l'ampiciline et deux séquences de recombinaison attP en orientation inverse séparées par un espaceur qui doit être au moins de 2kb.

Phagemide pM867 (6.5 kb) : Vecteur possédant l'origine de réplication "bas nombre de copies" p15A, l'intégrase du phage lambda ainsi que le répresseur thermosensible cI857, le marqueur de sélection à la kanamycine, et deux séquences de recombinaison attB en orientation inverse séparées par les séquences suivante : une unité de transcription pour les régions variables de chaînes lourdes (LacZ-pelB-VH), l'origine de réplication du phage F1, le promoteur tryptophane Trp suivi de l'antiterminateur NutL dans la même orientation.

Après recombinaison (2 événements de recombinaison), il y a échange des séquences séparant les séquences de recombinaison entre le plasmide et le phagemide de départ. Un phagemide recombinant est obtenu avec deux séquences attR en orientation inverse et séparées par les éléments du plasmide de départ, soit l'unité de transcription des régions variables de chaînes lourdes, l'origine F1, le promoteur Trp suivi de NutL. Dans ce phagemide recombinant possédant les deux unités de transcription pour les chaînes lourde et légère, il y a aussi création d'une unité de transcription pour un nouveau marqueur de sélection, ici la gentamycine (promoteur Trp-NuTL-N-gentamycine).

Les autres propriétés de ce système (non-reversibilité, nouveau marqueur de sélection après recombinaison), sont identiques à celle du système précédent décrit dans WO 95/21914.

### I - Description des différentes étapes de construction des deux vecteurs de départ.

I. Création d'un plasmide possédant une origine de réplication ColE1, un gène de résistance à l'ampicilline, un gène de résistance à la gentamycine (sans son promoteur), le gène de la protéine N, la cassette de clonage des chaînes légères et 2 séquences de recombinaison attP encadrant un espaceur d'environ 2 Kb.
   Ce vecteur servira de point de départ pour insérer la banque de chaînes légères (régions variables).
   1. Insertion de la 1ère séquence de recombinaison attP (271 pb) après amplification par PCR sur le phage λ (des bases 27571 à 27820), entre les sites EcoRI et KpnI du plasmide pUC18 (2686 pb) (Orientation contrôlée).
      On obtient le plasmide pM 852 (2957 pb). 
Amorce AttP Eco-Nhe+ : 5'GGAATTCCGGCTAGCCGCGCTAATGCTCTGTTACAG3'
EcoRI SphI
 
Amorce AttP Kpn-: 5' GGGGTACCCCATCAAATAATGATTTTATTT 3'
KpnI
   2. Insertion du gène de résistance à la gentamycine (gène aacC1) couplé à la seconde séquence de recombinaison attP.
      - La séquence attP est amplifiée par PCR à partir du phage λ des bases 27571 à 27820 avec les amorces suivantes : 
Amorce AttP Xba-Nhe+ : 5′ GCTCTAGAGGGCTAGCGCTAATGCTCTCTGTTACAG 3′
XbaI NheI
 
Amorce AttP Bam- : 5' CGGGATCCCATCAAATAATGATTTTATTT 3'
BamHI
      - Le gène aacC1 de 732 pb est amplifié à partir du plasmide pSS11 9 (réf: STIBITZ S., Methods in enzymology, Vol 235, 458-465, 1994). 
Amorce Genta 3' : 5' TTGGCGCGCCGAATTGTTAGGTGGCGGTACTTG 3'
AscI
 
Amorce AttP-Genta5' : 5'AAAATCATTATTTGATGGGATCCTAAGCCTGTTCGGTTCG-
TAAAC 3'
      - Une seconde amplification par PCR permet de réunir les 2 séquences précédentes grâce aux amorces suivantes : 
Amorce AttP Xba-Nhe+ : 5′ GCTCTAGAGGGCTAGCGCTAATGCTCTCTGTTACAG 3′
XbaI NheI
 
Amorce Genta 3' : 5' TTGGCGCGCCGAATTGTTAGGTGGCGGTACTTG 3'
AscI
         Le fragment comportant le gène aacC1 et la séquence attP, digéré par XbaI, est cloné dans le vecteur pM852 au niveau des sites Smal et XbaI avec destruction du site SmaI (Orientation contrôlée).
         On obtient le plasmide pM854 (3960 pb).
   3. Délétion d'une partie de la séquence encadrant le gène de la β-galactosidase dans le plasmide pM854 par digestion avec HindIII et SspI, puis traitement des extrémités du vecteur à la Klenow et ligation du plasmide sur lui-même avec destruction des 2 sites de restriction.
      On obtient le plasmide pM855 (3378 pb).
   4. Le site de restriction XbaI est détruit par digestion du vecteur pM855 par Xba1 et traitement de ses extrémités par la Klenow.
      On obtient le plasmide pM856 (3382 pb).
   5. Insertion d'une cassette d'expression de la chaîne légère (promoteur lac, séquence signal PelB et la chaîne légère (642 pb) d'un clone anti-gp 160 d'HIV fusionnée à gène III), couplée au système apportant une nouvelle résistance au phagemide recombiné (gène de la protéine N et gène aacC1 dépourvu de son promoteur).
      Le fragment complet (2609 pb) est obtenu à partir du plasmide pM845 (décrit ci-après) par digestion complète par SphI et digestion partielle par Asp718. Le fragment après traitement de ses extrémités à la Klenow est cloné dans le plasmide pM856 coupé par SphI et traité lui aussi à la Klenow, avec destruction du site phI.
      On obtient le plasmide pM857 (5991 pb).
   6. Le plasmide pM857 est délété du gène aacC1 par coupure KpnI-BamHI (768 pb), puis ses extrémités sont traitées à la Klenow. Un espaceur d'environ 2 Kb sera inséré entre ces deux sites. La séquence de cet espaceur reste encore à déterminer.
      On obtient le plasmide pM858 (environ 7200 pb).
II. Création d'un vecteur type phagemide portant 2 origines de réplication P15A et f1, un gène de résistance à la kanamycine, deux séquences de recombinaison attB encadrant la cassette de clonage des chaînes lourdes, ainsi que le promoteur Tryptophane et la séquence nutL.
   Ce vecteur servira de point de départ pour insérer une banque de chaîne lourdes (régions variables).
   Le plasmide de base pour la construction est le plasmide pM825 (décrit dans la demande WO 95/21914).
   1. Insertion d'une seconde séquence de recombinaison attB de 23 pb sous forme d'oligonucléotides synthétiques dans le site SphI. Les deux orientations possibles de clonage ont été obtenues. L'orientation qui nous intéresse est celle des 2 attB en sens opposés.
      On obtient le plasmide pM851 (3079 pb). Amorce AttB Sph+ : 5′ CCTGCTTTTTTATACTAACTTGCATG 3′ Amorce AttB Sph- : 3' GTACGGACGAAAAAATATGATTGAAC 5'
   2. Un fragment de 2118 pb (couvrant les bases 997 à 3079 + 0 à 36) du pM851 est amplifié par PCR. Il comprend les deux séquences de recombinaison attB, le gène de résistance à la kanamycine et l'origine de réplication P15A avec création d'un site AscI à chaque extrémité et élimination des sites EcoRI et SphI existants.
      On obtient le plasmide pM861 (2126 pb). 
Amorce pM851 AscI+ :
5'TTGGCGCGCCCAAGTTAGTATAAAAAAGCAGGCAGCTCGAACTCCCCTTAATAA 3'
AscI
 
Amorce pM851 AscI- :
5' TTGGCGCGCCAAGTTAGTATAAAAAAGCAGCCTGCCGACTTCCATTCAACAA 3'
AscI
   3. Mutagénèse du site de restriction XhoI situé en position 925 avec le kit Clontech.
      On obtient le plasmide pM862 (2126 pb). Amorce Seq pac- : 5' GTTTTCAGAGCAAGAGA 3' 
Amorce Oligo mut Xho : 5′ P-ATCGCGGCCTTGAGCAAGACG 3′
XhoI détruit
   4. Insertion d'un site multiple de clonage "polylinker" synthétique de 36 pb comprenant les sites XbaI, NcoI, SphI, et EcoRI au niveau du site AscI unique du vecteur pM862 (Orientation contrôlée).
      On obtient le plasmide pM863 (2162 pb). Amorce Poly1 : 5′ CGCGCCTCTAGACGCCATGGCATGCGCGAATTCGG 3′ 
Amorce Poly2 : 3' ____GGAGATCTGCGGTACCCGTACGCGCTTAAGCCGCGC 5'
AscI XbaI NooI SphI EcoRI AmcI
   5. Insertion dans le vecteur pM863 d'un fragment de 1122 pb comprenant la séquence nutL, la séquence du promoteur Tryptophane et l'origine de réplication f1. Ce fragment est extrait du vecteur pM847 (décrit ci-après) par digestion par NheI et BspHI, puis cloné dans le vecteur pM863 au niveau des sites XbaI et NcoI (orientation contrôlée).
      On obtient le phagemide pM864 (3284 pb).
   6. Insertion dans le vecteur pM864 d'un fragment de 1080 pb correspondant à la cassette d'expression des chaînes lourdes (promoteur lac, séquence signal PelB et la chaîne lourde (684 pb) d'un clone anti-gp160 d'HIV).
      Ce fragment est extrait du vecteur pM847 par digestion SphI + EcoRI, puis cloné dans le vecteur pM864 au niveau des sites SphI et EcoRI (Orientation contrôlée).
      On obtient le phagemide pM865 (4363 pb).
   7. Insertion du gène de l'intégrase de λ et de son promoteur amplifié par PCR (1280 pb) à partir du plasmide pCW107 (Wülfing & Plückthun, Gene 136, 199-203 : 1993). Ce fragment sera inséré au niveau des sites NheI et StuI du phagemide pM865. (Orientation contrôlée).
      On obtient le phagemide pM866 (environ 5643 pb).
   8. Insertion de la séquence cI857 sous la dépendance du promoteur pR de λ, amplifié par PCR (820 pb) à partir du plasmide pCW107. Cette séquence est nécessaire pour permettre l'expression de l'intégrase après un choc thermique à 42°C. Ce fragment sera inséré au niveau des sites AvaII et BspHI du phagemide pM866.
      On obtient le phagemide pM867 (environ 6463 pb).

Les deux dernières étapes 7 et 8 sont susceptibles d'être modifiées.

La construction de ces deux vecteurs permet d'utiliser n'importe quelle souche bactérienne pour la recombinaison (type XL1 blue).

### II Description des plamides pM845 et pM847 utilisés pour la construction des vecteurs de départ.

I. Construction du plasmide pM845 possédant une origine de réplication ColE1, un gène de résistance à la kanamycine, une séquence de recombinaison attB et une cassette permettant le clonage et l'expression des chaînes légères fusionnées au produit du gène III.
   1. Clonage du gène aacC1 (Gm^{R}) dans le plasmide pM826.
      - Amplification par PCR du gène aacC1 (575 pb) dépourvu de son promoteur à partir du pSS1129 (Stibitz S., Methods in enzymology, Vol 235, 458-465, 1994) et insertion au niveau du site unique AscI du pM826 décrit dans la demande WO 95/21914.
         On obtient le plasmide pM840 (3611 pb). 
Amorce Genta 5' :
5' TTGGCGCGCCAGGAGCTATGGAGCAGCAACGATGTTACGCAGCA 3'
AscI
 
Amorce Genta 3' :
5' TTGGCGCGCCGAATTGTTAGGTGGCGGTACTTG 3'
AscI
   2. Changement de l'origine de réplication P15A du plasmide pM840 par l'origine haut nombre de copies ColEl.
      - Amplification par PCR de ColE1 (953pb) à partir du vecteur pM840 dépourvu de l'origine P15A (2750 pb). 
Amorce ColE1 Mlu+ : 5' TTCGACGCGTCTGTCAGACCAAGTTTACTC 3'
MluI
 
Amorce ColE1 Bam- : 5' CGGGATCCTCTTCCGCTTCCTCGCTCA 3'
BamHI
 
Amorce AttB Bam+ : 5′ CGGGATCCGGAATTCGAGCTGCCTGCT 3′
BamHI
 
Amorce Kan Mlu- : 5' TTCGACGCGTAGGCCTGAATCGCCCCATCA 3'
MluI
      - Assemblage des deux fragments PCR grâce aux sites MluI et BamHI nouvellement créés.

      On obtient le plasmide pM842 (3692 pb).
   3. Clonage de gène III en 3' de la chaîne légère dans le plasmide pM842.
      - Amplification par PCR de gène III (658 pb) à partir du phagemide pM841 et insertion en 3' de la chaîne légère en conservant le cadre de lecture (avec un codon Ambre entre les deux) entre les sites XbaI et SphI.
         On obtient le plasmide pM844 (4339 pb). 
Amorce GeneIII Xba+ : 5' GCTCTAGAGTGGTGGCGGTGGCTCT 3'
XbaI
 
Amorce GeneIII Sph- : 5' ACACATGCATGCTTAAGACTCCTTATTACG 3'
SphI
   4. Clonage de la protéine N (nécessaire au fonctionnement de l'antiterminateur nutL) en 3' d'attB et en 5' du gène aacC1.
      - Amplification par PCR du gène de la protéine N (453 pb) à partir du phage lambda (bases 35022 à 35465) et du gène aacC1 (575 pb) à partir de pSS1129 puis association des deux fragments grâce à une seconde PCR avec les amorces 5' N Mlu et Genta 3'. 
Amorce 5'N NluI : 5' TTCGACGCGTAAGTGCGATTCCGGATTAGC 3'
MluI
 
Amorce 3'N BglII : 5' GAAGATCTAATATCTAAGTAACTAGAT 3'
BglII
 
Amorce N Genta 5'BglII :
5' ATCTAGTTACTTAGATATTAGATCTTCAGAGCTATGGAGCAGCAACGAT 3'
BglII
 
Amorce Genta 3' : 5' TTGGCGCGCCGAATTGTTAGGTGGCGGTACTTG 3'
AscI
      - Insertion du fragment PCR de 1022 pb au site AscI de pM844 après délétion du gène aacC1 précédemment cloné.

      On obtient le plasmide pM845 (4785 pb).
II. Construction du phagemide pM847 possédant une origine de réplication P15A, un gène de résistance à l'ampicilline une séquence de recombinaison attP et une cassette pour le clonage et l'expression des chaînes lourdes.
   1. Clonage de l'antiterminateur nutL dans le phagemide pM834 (décrit dans WO 95/21914).
      - Destruction du site NotI situé en 3' du promoteur lac par digestion puis remplissage à la Klenow.
      - Synthèse de 4 oligonucléotides recouvrant les 120 pb de la séquence nutL du phage lambda (bases 35467 à 35586) et insertion au niveau du site NotI situé en 3' de la séquence de recombinaison attP. Une seule orientation est valable pour que l'antiterminateur fonctionne après recombinaison.
         On obtient le phagemide pM839 (4937 pb). 
Amorces nutL1, nutL2, nutL3 et nutL4 :
5' GGCCGCACATCAGCAGGACGCACTGACCACCATGAAGGTGACGCTCTTAAAAATTAAGCCC-
TGAAGAAG
3' ____CGTGTAGTCGTCCTGCGTGACTGGTGCTACTTCCACTGCGAGAATTTTTAATTCGGG-
NotI
ACTTCTTC

GGCAGCATTCAAAGCAGAAGGCTTTGGGGTGTGATACGAAACGAAGCATTGGC 3'
CCGTCGTAAGTTTCGTCTTCCGAAACCCCACACACTATGCTTTGCTTCGTAACCGCCGG 5'
NotI
   2. Changement de l'origine de réplication ColE1 dans le phagemide pM839 par l'origine faible nombre de copies P15A.
      - Amplification par PCR de P15A (833 pb) à partir du pM840 et du vecteur pM839 dépourvu de l'origine Co1E1 (4007pb). 
Amorce P15A Xb+ : 5′ AATCTAGAGGAGTGTATACTGGCTAA 3′
XbaI
 
Amorce P15A Sph- : 5′ AGGCGCATGCTTAATAAGATGATCTTCTTG 3′
SphI
 
Amorce Pro Lac Sph+ : 5' ACACATGCATGCGCCCAATACGCAAACCG 3'
SphI
 
Amorce Amp Xba- : 5' GCTCTAGATTACCAATGCTTAATCAG 3'

XbaI
      - Assemblage des deux fragments PCR grâce aux sites XbaI et SphI nouvellement créés.

      On obtient le phagemide pM841 (4828 pb).
   3. Délétion du gène III situé en 3' de la chaîne lourde
      - Amplification par PCR du phagemide pM841 en entier mais sans gène III (4150 pb) et ligation après digestion par le site unique SpeI. On obtient le phagemide pM846 (4144 pb). 
Amorce Amb PCR : 5' GCTCTAGACTAACTAGTTTGTCACAAGATTTG 3'
SpeI
 
Amorce AttP Eco/Spe- : 5' GGACTAGTTAAGAATTCATCAAATAATGATTTTA 3'
SpeI EcoRI
   4. Changement du promoteur cat par le promoteur trp.
      - Amplification par PCR du promoteur tryptophane bicistronique (233 pb) à partir du vecteur pM800 (disponible dans le laboratoire) et insertion au niveau du site KpnI situé en 3' de la séquence nutL après délétion du promoteur cat précédemment cloné. On obtient le phagemide pM847 (4194 pb).

Amorce Pro Trp Mut Kpn+ :
5' CGGGGTACCACAATTAATCATCGAACAAGTTATCTAGTACGCA 3'
KpnI

Amorce Pro Trp Kpn- : 5′ GGGGTACCTTTAAAGTCGGTTTTTGT 3′
KpnI

### III - Recombinaison entre les deux vecteurs pM858 et pM867.

Les deux vecteurs précités serviront de point de départ pour obtenir des banques d'anticorps multicombinatoires. Dans l'exemple représenté la recombinaison s'effectuera entre les chaînes V_{L} et V_{H} du clones anti-gp 160 de HIV utilisé. Cependant si les deux vecteurs sont construits à partir de banques de gènes de chaînes lourdes et/ou de gènes de chaînes légères d'anticorps ils permettront d'obtenir une banque multicombinatoire d'anticorps que l'on pourra ensuite cribler.

Transformés dans une souche adéquate (D1210HP), les deux vecteurs pourront s'associer grâce aux séquences AttP et AttB, cibles d'un facteur de recombinaison int inductible. Les mécanismes de recombinaison sont décrits dans le chapitre 9 du livre "The recombination of genetic material" (Miller H. V., Viral and cellular control of site-specific recombination, 1988, p 360-384, edited by Brooks Low K., Academic Press). Le vecteur multicombinatoire (7 200 pb) ainsi créé possèdera une origine de réplication, un gène de résistance à la gentamycine et les deux cassettes permettant l'expression des chaînes V_{L} et V_{H}.
1) On procède à la transformation par électroporation de la souche de E. coli XL-1 Blue (diffusée par Stratagène).
2) Etapes de la recombinaison.
   - On procède à la transformation de cette souche par le plasmide pM 858 (contenant les chaînes V_{L}), et en parallèle on transforme une souche compatible, par le phagemide pM867 (portant les chaînes VH).

A partir de la souche transformée par le phagemide pM 867 on prépare celui-ci sous forme de phage puis on infecte la culture de XL-1 Blue contenant le plasmide pM 858, à 30°C (densité optique DO = 0,6).

Après 30 mn d'infection à 30°C, on soumet la culture à un choc thermique à 42°C pendant une heure pour déclencher la recombinaison sous l'effet de la recombinase inductible.

Après avoir remis la culture à 30°C et ajouté de la gentamycine on étale les clones sur milieu gélosé contenant de la gentamycine. On ajoute une heure après le phage helper VCSM13 (Kan^{R}) de Stratagène à raison de 10¹² pfu/100 ml de culture. Puis on ajoute, deux heures après, la kanamycine à 70 µg/ml final et la culture est laissée quelques heures à 30°C.

L'analyse des clones après recombinaison des vecteurs pM858 et pM867 montre que l'association s'est bien déroulée. On obtient un phagemide recombiné de 7 200 pb stable, exprimant un Fab et toujours capable d'infecter la souche XL-1 Blue. Les points de jonction AttL et AttR ont été vérifiés par séquençage.

### IV - Préparation d'une banque multicombinatoire à partir d'une banque de chaînes légères et d'une banque de chaînes lourdes.

L'étape d'insertion I.4 de la région variable de la chaîne légère du clone anti-gp 160 de HIV amplifié par PCR est remplacé par une insertion similaire des régions variables des chaînes légères d'une banque de chaînes légères d'anticorps amplifiée par PCR à l'aide d'un système d'amorces connu convenable propre au type de chaîne légère recherchée, ou d'une pluralité de systèmes d'amorces si l'on souhaite effectuer la multicombinaison à partir de populations de différents types de chaînes légères. Les systèmes d'amorce permettant l'amplification des chaînes légères sont bien connus et décrits par W.D. Huse et al., Science, Vol 246 (1989), 1275-1281.

De la même façon, pour le clonage des chaînes lourdes, on remplace l'étape d'insertion II.1 de la chaîne lourde d'un clone anti-gp 160 HIV par l'insertion d'une banque de régions variables de chaînes lourdes amplifiée par PCR à l'aide des systèmes d'amorce convenables décrits par M.J. Campbell et al., Molecular Immunology, Vol. 29, No. 2 (1992), 193-203 ou par W.D. Huse et al. ci-dessus.

Après les étapes de recombinaison les clones résistant à la gentamycine sont sélectionnés et on procède ensuite au criblage destiné à sélectionner les clones exprimant des associations de chaîne légère et chaîne lourde d'anticorps ayant les affinités recherchées contre les antigènes déterminés, conformément aux techniques décrites dans par exemple par S.F. Parmley et al., Gene 73 (1988), 305-318.

Les clones issus des étapes de sélection et de criblage permettent ensuite de produire les molécules Fab correspondantes. Le phagemide utilisé contient un codon ambre à la jonction de la chaîne lourde du Fab et de la gp3. Lorsqu'à titre d'exemple, une souche d'E. coli suppressive d'ambre, XL1-Blue (Stratagène, La Jolla, CA, EU), est transformée avec le phagemide, le Fab est exprimé à la surface du phage. A titre d'exemple, dans une souche bactérienne non suppressive, TOP10 (Statagène, la Jolla, CA, EU), la synthèse protéique est interrompue à l'extrémité de la chaîne lourde. Grâce à la séquence signal du gène codant pour la pectase lyase "pelB" d'E. *cartovora*, le Fab est alors transporté dans le périplasme. Il est ensuite extrait par choc osmotique (Neu, H.C et al. (1965). J. Biol. Chem. 240, 3685-3692). En fonction de la nature du Fab, différentes techniques chromatiques connues peuvent être employées pour le purifier : par échange d'ions, par affinité, tamis moléculaire...

## Revendications

1. Procédé de production de banques multicombinatoires dans lequel, partant d'un premier répertoire de gènes codant pour une population d'un de deux types de polypeptides susceptibles de s'associer, de manière covalente ou non, notamment de régions variables de l'un des types chaîne légère et chaîne lourde d'anticorps, et d'au moins d'un gène codant pour l'autre type de polypeptide, notamment une région variable de l'autre type de chaîne d'anticorps ou de préférence d'un second répertoire de gènes codant pour une population dudit autre type,
- on introduit les gènes dudit premier répertoire dans un premier vecteur de départ pour former une population de vecteurs finaux portant les différents gènes dudit premier répertoire,
- et on introduit ledit gène dudit autre type ou les gènes dudit second répertoire dans un second vecteur de départ,
lesdits vecteurs de départ contenant respectivement deux sites attP du phage lambda et deux sites attB de E. coli en orientation inverse de façon à permettre deux événements de recombinaison sous l'effet d'une recombinase ou intégrase associée pour former dans chacun des vecteurs finaux résultant de la recombinaison, deux séquences de jonctions stables attL et attR,
- et l'on procède à deux événements de recombinaison irréversible,
de façon à générer, après recombinaisons, des vecteurs finaux recombinés dont l'un contient un gène de l'un des deux types et un gène de l'autre type, ledit vecteur permettant d'exprimer les deux gènes à la surface d'un phage sous forme de polypeptides maintenus et associés entre eux de façon multimérique ou simulant un multimère.

2. Procédé selon la revendication 1, dans lequel l'un des vecteurs de départ comprend une séquence codant pour une recombinase ou une intégrase.

3. Procédé selon l'une des revendications 1 ou 2, dans lequel le vecteur final recombinant obtenu à partir desdits vecteurs de départ et qui contient les gènes à exprimer est agencé pour présenter un marqueur de sélection initialement non fonctionnel et rendu fonctionnel par la recombinaison.

4. Procédé selon la revendication 3, dans lequel le marqueur de sélection comporte un gène permettant la sélection, lorsqu'il est exprimé, et un promoteur propre au gène, le promoteur étant inséré dans l'un des vecteurs de départ et le gène marqueur dans l'autre.

5. Procédé selon la revendication 4, dans lequel le gène de sélection et son promoteur sont séparés par une séquence antiterminatrice, notamment la séquence NutL.

6. Procédé selon l'une des revendications 3 à 5, dans lequel le marqueur est le gène de résistance à un antibiotique, notamment un gène du groupe formé par les gènes de résistance aux tétracyclines, à la genamycine, à la kanamycine et au chloramphénicol, avec son promoteur.

7. Procédé selon l'une des revendications 1 à 6, dans lequel lesdits vecteurs de départ contiennent au moins une origine de réplication de phage ou une origine de réplication de plasmide, lesdites origines étant agencées de manière à ce que les vecteurs finaux recombinants ne contiennent chacun qu'une origine de réplication de phage et/ou de plasmide.

8. Procédé selon l'une des revendications 1 à 7, dans lequel l'un des gènes à exprimer est fusionné à une séquence codant pour tout ou partie d'un polypeptide de la capside d'un phage.

9. Procédé selon la revendication 8, dans lequel la séquence de fusion correspond au gène codant pour la protéine III du phage lambda.

10. Procédé selon l'une des revendications 8 et 9, dans lequel ledit gène et sa séquence fusionnée sont situés dans un des vecteurs de départ de manière à être présents dans le produit phagemide recombinant.

11. Procédé selon l'une des revendications 1 à 10, dans lequel on utilise pour contrôler l'étape de recombinaison entre les vecteurs de départ, une recombinase inductible et notamment thermo-inductible.

12. Vecteur susceptible d'être obtenu par le procédé selon l'une des revendications 1 à 11, et notamment vecteur du type plasmide ou phagemide, comprenant une séquence codant pour l'un des deux types de polypeptides susceptibles de s'associer, notamment une partie variable de chaîne légère d'anticorps, et une séquence codant pour l'autre desdits deux types, notamment une partie variable de chaîne lourde d'anticorps, lesdites séquences étant accompagnées, dans des cadres convenables, des éléments permettant leur expression dans un hôte, lesdites séquences étant séparées par les séquences de jonction stables attR et attL.

13. Vecteur selon la revendication 12, comprenant des séquences de jonction stables séparées par un espaceur.

14. Banques multicombinatoires de vecteurs, formées par les vecteurs selon l'une des revendications 12 et 13 et associant, de façon aléatoire, une séquence codant pour l'un des deux types de polypeptides susceptibles de s'associer, notamment une partie variable de chaîne légère d'anticorps et une séquence codant pour l'autre desdits deux types, notamment une partie variable de chaîne lourde d'anticorps.

## Claims

1. Method for the production of multicombinatorial libraries, wherein, starting from a first repertoire of genes coding for a population of one of two types of polypeptides, which can combine covalently or otherwise, in particular of variable regions of one of the antibody light and heavy chain types, and at least of one gene coding for the type of polypeptide, in particular a variable region of the other antibody chain type or preferably of a second repertoire of genes coding for a population of said other type,
- the genes of said first repertoire are introduced into a first starting vector to form a population of final vectors carrying different genes from said first repertoire,
- and said gene of said other type or genes of said second repertoire are introduced into a second starting vector,
said starting vectors respectively containing two attP lambda phage sites and two attB E. coli sites in opposite orientation, so as to allow two recombination events under the influence of an associated recombinase or integrase to form two stable attL and attR attachment sequences, in each of the final vectors resulting from recombination,
- and two events of irreversible recombination are performed,
in order to generate, after the recombinations, final recombinant vectors, one of which contains a gene of one of the two types and a gene of the other type, said vector allowing the two genes to be displayed on the surface of a phage in the form of polypeptides, remaining there and being combined together multimerically or in a manner simulating a multimer.

2. Method according to Claim 1, wherein one of the starting vectors comprises a sequence coding for a recombinase or integrase.

3. Method according to one of Claims 1 or 2, wherein the final recombinant vector, which is obtained from said starting vectors and which contains the genes to be expressed, is arranged to have a selection marker that is initially non-functional and is rendered functional by recombination.

4. Method according to Claim 3, wherein the selection marker comprises a gene enabling selection when it is expressed and a promoter for the gene, said promoter being inserted in one of the starting vectors and the marker gene in the other.

5. Method according to Claim 4, wherein the selection gene and its promoter are separated by an antiterminating sequence, in particular the NutL sequence.

6. Method according to one of Claims 3 to 5, wherein the marker is an antibiotic resistance gene, in particular a gene of the group consisting of genes resistant to tetracyclines, to genamycin, to kanamycin and to chloramphenicol, with its promoter.

7. Method according to one of Claims 1 to 6, wherein said starting vectors contain at least one phage origin of replication or one plasmid origin of replication, said origins being arranged such that the final recombinant vectors each only contain one phage and/or plasmid origin of replication.

8. Method according to one of Claims 1 to 7, wherein one of the genes to be expressed is fused to a sequence coding for all or part of a polypeptide of the capsid of a phage.

9. Method according to Claim 8, wherein the fusion sequence corresponds to the gene coding for the III protein of the lambda phage.

10. Method according to one of Claims 8 and 9, wherein said gene and its fused sequence are situated in one of the starting vectors so as to be present in the recombinant phagemid product.

11. Method according to one of Claims 1 to 10, wherein an inducible and in particular thermo-inducible recombinase is used to control the recombination step between the starting vectors.

12. Vector capable of being obtained by the method according to one of Claims 1 to 11, and in particular a vector of the plasmid or phagemid type, comprising a sequence coding for one of the two types of polypeptides, which can combine together, in particular for a variable portion of the antibody light chain, and a sequence coding for the other of said two types, in particular for a variable portion of the antibody heavy chain, said sequences being accompanied in suitable frames by elements that allow their expression in a host, said sequences being separated by the stable attR and attL attachment sequences.

13. Vector according to Claim 12, comprising stable attachment sequences separated by a spacer.

14. Multicombinatorial libraries of vectors formed by the vectors according to one of Claims 12 and 13, combining in a random manner a sequence coding for one of the two types of polypeptides, which can combine together, in particular for a variable portion of the antibody light chain and a sequence coding for the other of said two types, in particular for a variable portion of the antibody heavy chain.

## Patentansprüche

1. Verfahren zur Herstellung multikombinatorischer Banken, in welchem, ausgehend von einer ersten Bibliothek von Genen, die für eine Gesamtheit aus einem von zwei Polypeptid-Typen, insbesondere variablen Regionen einer kleinen oder großen Antikörper-Kette, die sich gegebenenfalls kovalent miteinander verbinden können, codieren, und mindestens einem Gen, das für den anderen Polypeptid-Typ, insbesondere eine variable Region des anderen Antikörperketten-Typs, codiert, oder vorzugsweise einer zweiten Bibliothek von Genen, die für eine Gesamtheit des anderen Typs codieren,
- die Gene der ersten Bibliothek in einen ersten Ausgangsvektor eingeführt werden, um eine Gesamtheit aus fertigen Vektoren zu bilden, welche die verschiedenen Gene der ersten Bibliothek tragen,
- das Gen für den anderen Typ oder die Gene der zweiten Bibliothek in einen zweiten Ausgangsvektor eingeführt wird/werden, wobei die Ausgangsvektoren jeweils zwei Loci attP des Lambda-Phagen und zwei Loci attB von *E. coli* mit umgekehrter Orientierung derart enthalten, dass sie zwei Rekombinationen unter dem Einfluß einer Recombinase oder Integrase ermöglichen, die gebunden ist, um in den fertigen Vektoren, die aus der Rekombination resultieren, je zwei stabile attL- und attR-Bindungssequenzen zu bilden, und
- zwei irreversible Rekombinationen derart durchgeführt werden, dass nach Rekombinationen fertige rekombinierte Vektoren erzeugt werden, wovon ein Vektor ein Gen für einen der beiden Typen und ein Gen für den anderen Typ enthält und es ermöglicht, die zwei Gene auf der Oberfläche eines Phagen in Form von Polypeptiden zu exprimieren, die wie ein multimeres Protein oder ein multimeres Protein vortäuschend festgehalten und miteinander verbunden werden.

2. Verfahren nach Anspruch 1, in welchem einer der Ausgangsvektoren eine Sequenz enthält, die für eine Recombinase oder Integrase codiert.

3. Verfahren nach Anspruch 1 oder 2, in welchem der rekombinante fertige Vektor, der aus den Ausgangsvektoren erhalten worden ist und die zu exprimierenden Gene enthält, erzeugt wird, um einen Selektionsmarker aufzuweisen, der anfänglich nicht funktionell war und durch die Rekombination funktionell gemacht worden ist.

4. Verfahren nach Anspruch 3, in welchem der Selektionsmarker ein Gen, das die Selektion ermöglicht, wenn es exprimiert wird, und einen dem Gen eigenen Promotor enthält, wobei der Promotor in einen der Ausgangsvektoren und das Markergen in den anderen insertiert wird.

5. Verfahren nach Anspruch 4, in welchem das Selektionsgen und dessen Promotor durch eine Antiterminationssequenz, insbesondere die Sequenz NutL, voneinander getrennt sind.

6. Verfahren nach einem der Ansprüche 3 bis 5, in welchem der Marker das Antibiotikaresistenz-Gen, insbesondere ein Gen der Gruppe, die von den Genen der Resistenz gegen Tetracycline, Gentamycin, Kanamycin und Chloramphenicol gebildet wird, mit seinem Promotor ist.

7. Verfahren nach einem der Ansprüche 1 bis 6, in welchem die Ausgangsvektoren mindestens einen Phagenoder Plasmidreplikationsursprung enthalten, wobei die Ursprünge derart erzeugt werden, dass die rekombinanten fertigen Vektoren jeweils nur einen Phagen- und/oder Plasmidreplikationsursprung enthalten.

8. Verfahren nach einem der Ansprüche 1 bis 7, in welchem eines der zu exprimierenden Gene mit einer Sequenz fusioniert wird, die für ein Polypeptid des Capsids eines Phagen oder einen Teil davon codiert.

9. Verfahren nach Anspruch 8, in welchem die Fusionssequenz dem Gen entspricht, das für das Protein III des Lambda-Phagen codiert.

10. Verfahren nach Anspruch 8 oder 9, in welchem sich das Gen und dessen fusionierte Sequenz derart in einem der Ausgangsvektoren befinden, dass sie in dem rekombinanten Phagemidprodukt vorhanden sind.

11. Verfahren nach einem der Ansprüche 1 bis 10, in welchem zur Kontrolle der Stufe der Rekombination zwischen den Ausgangsvektoren eine insbesondere thermoinduzierbare induzierbare Recombinase verwendet wird.

12. Vektor, der durch das Verfahren nach einem der Ansprüche 1 bis 11 erhalten werden kann, insbesondere ein Vektor vom Typ Plasmid oder Phagemid, der eine Sequenz, die für einen der zwei Polypeptid-Typen, die sich miteinander verbinden können, insbesondere einen variablen Teil der kleinen Antikörperkette, codiert, und eine Sequenz, die für den anderen dieser zwei Typen, insbesondere einen variablen Teil der großen Antikörperkette, codiert, enthält, wobei die Sequenzen in geeigneten Rahmen von Elementen, die ihre Expression in einem Wirt ermöglichen, umgeben und durch die stabilen attR- und attL-Bindungssequenzen voneinander getrennt sind.

13. Vektor nach Anspruch 12, der durch einen Abstandshalter getrennte stabile Bindungssequenzen enthält.

14. Multikombinatorische Vektorbanken, die von Vektoren nach Anspruch 12 oder 13 gebildet werden, die eine Sequenz, die für einen der zwei Polypeptid-Typen, die sich miteinander verbinden können, insbesondere einen variablen Teil der kleinen Antikörperkette, codiert, mit einer Sequenz, die für den anderen der beiden Typen, insbesondere einen variablen Teil der großen Antikörperkette, codiert, auf eine zufällige Weise miteinander verknüpfen.
